# EUROPEAN PATENT APPLICATION

(11) **EP 4 083 184 A1**
(43) Date of publication of application: **02.11.2022**
(21) Application number: 21382360.2
(22) Date of filing: 26.04.2021
(51) Int. Cl.: C12M 1/22, C12M 1/34

(54) **DEVICE FOR OBTAINING IMAGES OF PETRI DISHES**

(71) Applicant: Sener Aeroespacial, S.A., 48930 Getxo-Vizcaya (ES)
(72) Inventor: ARIÑO BRINGUÉ, Juan, 48930 Getxo - Vizcaya (ES)
(74) Representative: Clarke Modet & Co.

(57) **Abstract**

The device for obtaining images of a Petri dish (9) characterised in that it comprises a hollow body (1) which in the preferred embodiment has the shape of a spherical cap with an opening (2) at one end, such that the hollow body (1) comprises a first camera (3) in the portion of the hollow body (1) furthest from the opening (2), at least one additional camera (4) between the first camera (3) and the opening (2), at least one lighting means in at least one perimeter channel (8), wherein the cameras (3, 4) are oriented towards the opening (2) which is the position wherein, outside the hollow body (1), the Petri dish (9) of which images are to be obtained is located.

## Description

### Field of the invention

The present invention relates to a device for obtaining images of Petri dishes, which enables images of Petri dishes to be taken with different lighting and different simultaneous camera positions, improving the devices known in the state of the art. The device object of the invention is applicable in microbiology laboratories.

### Background of the invention

Microbiology laboratories use conventional methods to observe the growth of bacteria in Petri dishes. In order to observe these bacteria, the sample is sown in said Petri dishes with an agar which facilitates growth under specific atmospheric conditions. Conventional methods for observing growth are based on a visual examination by the physician who performs the diagnosis based on the growth observed in the Petri dishes.

To perform the diagnosis, there are two methods depending on the type of analysis to be performed, qualitative or quantitative. The qualitative analysis consists of identifying colonies of certain bacteria, while the quantitative analysis consists of knowing the bacterial concentration had by the sample depending on the number of colony-forming units which grow on the dish.

At present, this process is being automated by means of optical systems which capture images that can also be reviewed by the physician or, in a more automated manner, processed by a computer capable of orienting the diagnosis and/or counting the colony-forming units.

In order to make the diagnosis using an image from the Petri dish itself, apart from the image quality, it is very important to achieve sufficient, homogeneous lighting without reflections.

Document EP3147348B1 is known wherein a sphere of lighting for Petri dishes is described, wherein only a single image sensor is defined which does not enable different images to be obtained from different angles as enabled by the present invention. Document EP3147348B1 defines a single lighting and does not propose using lighting with different wavelengths.

Document US5545561 is also known wherein a lateral, lower lighting system of the dish is described. Document US5545561 describes a Petri dish recognition system based on an image sensor installed in the lower portion. This system is based on maximising the contrast between the colony and the agar for the counting of colonies.

### Description of the invention

The object of the invention is a device for obtaining images of a Petri dish characterised in that it comprises a hollow body with an opening at one end, such that the hollow body comprises a first camera in the portion of the hollow body furthest from the opening and at the least one lighting means in at least one perimeter channel. In the device the object of the invention, the camera is oriented towards the opening, which is the position wherein, outside the hollow body, the Petri dish of which images are to be obtained is located. As for the perimeter lighting, around the Petri dish, the present invention achieves uniform lighting without reflections or dark areas.

The device for obtaining images of a Petri dish object of the invention can comprise at least one additional camera between the first camera and the opening, said camera also being oriented towards the opening, which is the position wherein, outside the hollow body, the Petri dish of which images are to be obtained is located.

In the preferred embodiment of the device for obtaining images of a Petri dish object of the invention, the hollow body has the shape of a spherical cap.

In the device for obtaining images of a Petri dish, the first camera is configured to obtain images of the Petri dish at an angle of 0° with respect to a vertical of the Petri dish, and wherein the additional camera is configured to obtain images of the Petri dish at an angle with respect to the vertical of the Petri dish.

In the device for obtaining images of a Petri dish object, the hollow body comprises a hole for each camera.

In the device for obtaining images of a Petri dish object of the invention, the hole of the hollow body has a size equal to the size of the lens of each camera.

In the device for obtaining images of a Petri dish object of the invention, the hollow body comprises a coating made of a material with a non-reflective finish therein which covers at least the surface between the additional cameras and the first camera creating two areas: a first dark area, non-reflective and light-absorbing, which prevents the camera from capturing the reflections which are produced on the base and walls of the dishes when the contents are translucent, and a second area which generates a diffuse reflection of light and lights the dish evenly.

In the device for obtaining images of a Petri dish object of the invention, it can comprise three perimeter channels with lighting means housed in each perimeter channel, such that each lighting means is configured to light up independently from the rest, such that the different lightings can be combined with each other in order to achieve different effects and enable the three-dimensional morphology of the colonies to be captured better.

In the device for obtaining images of a Petri dish object of the invention, each lighting means can have a different wavelength in both the visible and infrared spectra, thus multispectral images can be obtained and the identification of the type of growth of the colonies is facilitated.

In the device for obtaining images of a Petri dish, the lighting means include lighting points equidistant with the opening, which reduces the appearance of dark areas.

The device of the present invention aims to, in addition to counting the colonies, enable the qualification of said colonies. The aim of the device object of the invention is to obtain images with sufficient quality to perform the identification and quantification of bacteria in a manual or automated manner in order to perform the diagnosis of the patient.

This invention has been designed so that it is compatible with Petri dishes of a wide variety and dimensions, as well as enabling an easy automation of the process of handling dishes and identifying bacteria.

### Brief description of the drawings

What follows is a very brief description of a series of drawings that aid in better understanding the invention, and which are expressly related to three embodiments of said invention that are presented by way of a non-limiting examples of the same.

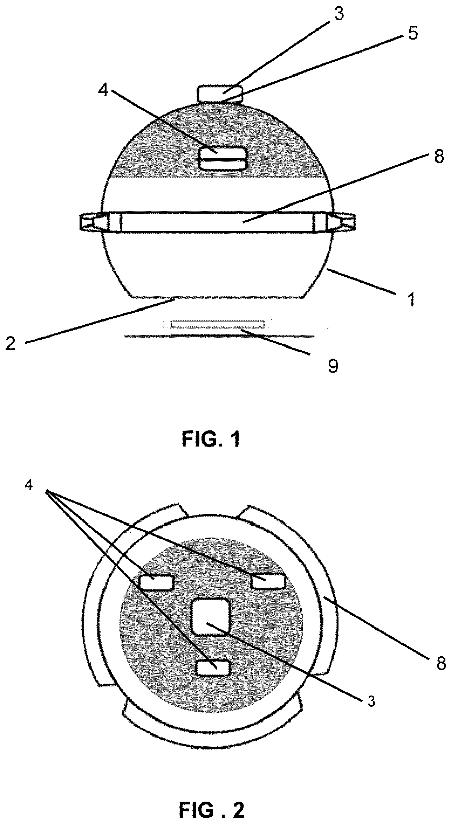

Figure 1 represents a side view of an embodiment of the device object of the invention.
Figure 2 represents a bottom view of the device object of the invention of Figure 1.

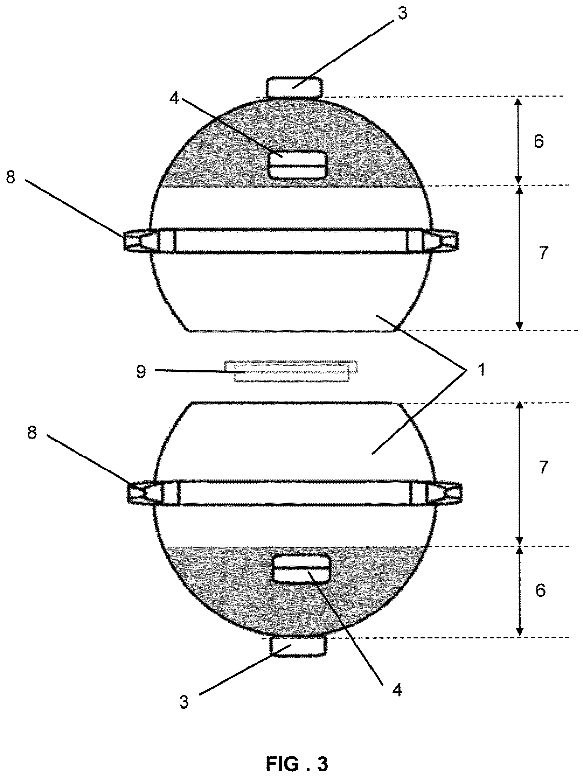

Figure 3 represents a side view of an alternative embodiment of the device object of the invention.

The numerical references used in the figures are:
1. hollow body,
2. opening,
3. first camera,
4. additional camera,
5. hole,
6. first dark area,
7. second area,
8. perimeter channel, and
9. Petri dish.

### Detailed description of the invention

The object of the invention is a device for obtaining images of Petri dishes (9) which has a hollow body (1) with an opening (2) at one end, such that the hollow body (1) houses four cameras (3, 4) on the outside thereof, distributed as follows, a first camera (3) in the portion of the hollow body (1) furthest from the opening (2) and three additional cameras (4) located between the first camera (3) and the opening (2), the three additional cameras (4) being located equidistant from each other.

In the hollow body (1), in correspondence with each of the cameras (3, 4) there is only one hole (5) with the size of the lenses of the camera (3, 4) such that the surface changes which can generate reflections in the images obtained by each camera (3, 4) are minimised.

All the cameras (3, 4) are oriented towards the opening (2) which is the position wherein, outside the hollow body (1), the Petri dish (9) of which images are to be obtained is located.

The first camera (3) enables images of the Petri dish (9) to be obtained at an angle of 0° with respect to the vertical of the same Petri dish (9). The three additional cameras (4) enable images to be obtained at an angle with respect to the vertical of the Petri dish (9). Likewise, each of them offers a 120° view of the Petri dish (9).

In the preferred embodiment of the invention, the hollow body (1) has the shape of a hollow spherical cap, said hollow spherical cap shape provides homogeneous lighting without reflection.

The hollow body (1) has therein a coating made of a material with a non-reflective finish such that the reflections which can be produced in the images due to the lenses of the cameras (3, 4) themselves are minimised, and which covers at least the surface between the additional cameras (4) and the first camera (3), thus creating two areas: a first dark area (6), non-reflective and light-absorbing, which prevents the camera from capturing the reflections which are produced on the base and walls of the dishes when the contents are translucent, and a second area (7) which generates a diffuse reflection of light and lights the dish evenly.

The device for obtaining images of Petri dishes (9) object of the invention further comprises a lighting system comprising three perimeter channels (8) located between the additional cameras (4) and the opening (2) of the hollow body (1).

In each of the perimeter channels (8), the device object of the invention houses lighting means which can be lighting means with different wavelengths, especially interesting in the visible, near infrared and mid-infrared spectra. This configuration enables different information about the growth of the Petri dish (9) to be obtained. As they are divided into three segments, different lighting configurations can be obtained in the same manner.

In the preferred embodiment of the invention, the one wherein the hollow body (1) is a spherical cap, the perimeter channels (8) are located in the area of the spherical cap with the largest radius.

The fact that the lighting means are located inside perimeter channels (8) prevents the light from being captured directly by the cameras (3, 4).

Each of the three lighting means can be switched on independently, enabling different lighting configurations for taking images from each of the cameras.

The spherical cap geometry is especially important for obtaining uniform lighting. All the lighting points are equidistant with the opening (2) and consequently with the Petri dish (9) located outside the opening (2), which contributes to obtaining uniform lighting on the surface of the Petri dish (9).

The device object of the invention can comprise two hollow bodies (1) facing each other by the corresponding openings (2) thereof, such that the Petri dish (9) is between the two hollow bodies (1) and images can be obtained of both faces of the Petri dish (9), in addition to enabling different lightings on the side of the Petri dish (9) opposite from the side of which the image is obtained.

## Claims

1. A device for obtaining images of a Petri dish (9) **characterised in that** it comprises a hollow body (1) with an opening (2) at one end, such that the hollow body (1) comprises:
- a first camera (3) in the portion of the hollow body (1) furthest from the opening (2),
- at least one lighting means in at least one perimeter channel (8),
wherein the camera (3) is oriented towards the opening (2) which is the position
wherein, outside the hollow body (1), the Petri dish (9) of which images are to be obtained is located.

2. The device for obtaining images of a Petri dish (9) according to claim 1, **characterised in that** it comprises at least one additional camera (4) between the first camera (3) and the opening (2), said additional camera (4) being oriented towards the opening (2).

3. The device for obtaining images of a Petri dish (9) according to any of claims 1 to 2, **characterised in that** the hollow body (1) has the shape of a spherical cap.

4. The device for obtaining images of a Petri dish (9) according to any of claims 2 to 3 **characterised in that** the first camera (3) is configured to obtain images of the Petri dish (9) at an angle of 0° with respect to a vertical of the Petri dish (9), and wherein the additional camera (4) is configured to obtain images of the Petri dish (9) at an angle with respect to the vertical of the Petri dish (9).

5. The device for obtaining images of a Petri dish (9) according to any of claims 2 to 4, **characterised in that** the hollow body (1) comprises a hole (5) for each camera (3, 4).

6. The device for obtaining images of a Petri dish (9) according to claim 5, **characterised in that** the hole (5) has a size equal to the size of the lens of each camera (3, 4).

7. The device for obtaining images of a Petri dish (9) according to any of claims 2 to 6, **characterised in that** the hollow body (1) comprises a coating made of a material with a non-reflective finish therein which covers at least the surface between the additional cameras (4) and the first camera (3) creating a first dark area (6), non-reflective and light-absorbing, and a second area (7) which generates a diffuse reflection of light.

8. The device for obtaining images of a Petri dish (9) according to any of claims 1 to 6, **characterised in that** it comprises three perimeter channels (8) with lighting means housed in each perimeter channel (8), such that each lighting means is configured to light up independently from the rest.

9. The device for obtaining images of a Petri dish (9) according to any of claims 1 to 8, **characterised in that** each lighting means has a different wavelength.

10. The device for obtaining images of a Petri dish (9) according to any of claims 1 to 9, **characterised in that** the lighting means comprise lighting points equidistant with the opening (2).

11. The device for obtaining images of a Petri dish (9) according to any of claims 1 to 10, **characterised in that** it comprises two hollow bodies (1) facing each other.
